# EUROPÄISCHE PATENTSCHRIFT

(11) **EP 1 143 926 B1**
(45) Veröffentlichungstag und Bekanntmachung des Hinweises auf die Patenterteilung: **25.05.2005**
(21) Anmeldenummer: 00903601.3
(22) Anmeldetag: 21.01.2000
(51) Int. Cl.: A61K 7/48

(54) **MIKROEMULSIONEN UND DEREN VERWENDUNG ZUR AUSRÜSTUNG SAUGFÄHIGER TRÄGERSUBSTRATE**
MICROEMULSIONS AND THEIR USE IN FINISHING ABSORBENT CARRIER SUBSTRATES
MICRO-EMULSIONS ET LEUR UTILISATION POUR DOTER DES SUBSTRATS DE SUPPORTS ABSORBANTS

(30) Priorität: 30.01.1999 DE 19903717
(43) Veröffentlichungstag der Anmeldung: 17.10.2001
(73) Patentinhaber: Henkel Kommanditgesellschaft auf Aktien, 40589 Düsseldorf (DE)
(72) Erfinder: FÖRSTER, Thomas, D-40699 Erkrath (DE); CLAAS, Marcus, D-40723 Hilden (DE); WADLE, Armin, D-40699 Erkrath (DE); MUNK, Gabriele, D-22763 Hamburg (DE)
(86) Internationale Anmeldenummer: PCT/EP2000/000450
(87) Internationale Veröffentlichungsnummer: WO 2000/044343

(56) Entgegenhaltungen:
- EP-A- 0 813 862
- EP-A- 0 842 606
- DE-A- 19 710 149
- DE-A- 19 710 155

## Beschreibung

Die Erfindung betrifft Mikroemulsionen mit einem Gehalt an öllöslichen oder wasserlöslichen kosmetischen oder dermatologischen Wirkstoffen und hautverträglichen Ölkomponenten, die sich zur Reinigung, Pflege oder zur dermatologischen Behandlung der Haut und zur Ausrüstung flexibler, saugfähiger Träger eignen.

Mikroemulsionen sind optisch isotrope, thermodynamisch stabile Systeme, die eine wasserunlösliche Ölkomponente, Emulgatoren und Wasser enthalten und die aufgrund ihrer sehr niedrigen Tröpfchengröße, die im wesentlichen unter 100 nm, mit Mittel immer unter 50 nm liegt, ein klares bzw. transparentes Aussehen haben.

Mikroemulsionen haben wegen ihrer gegenüber Makroemulsionen höheren Stabilität, feineren Verteilung der inneren Phase, der meist höheren Effektivität und der besseren transdermalen Penetration der darin eingearbeiteten Wirkstoffe eine erhebliche Bedeutung bei der Formulierung kosmetischer und pharmazeutischer Zubereitungen.

Aus DE 195 47 986 Cl waren O/W-Mikroemulsionen bekannt, die unter Verwendung von Fettsäure-N-polyhydroxyalkylamiden oder Alkyl-(oligo)-glucosiden als Emulgatoren und Fettsäure- oder Fettalkohol-Estern als Ölkomponenten erhalten wurden. Diese Emulsionen weisen zwar Teilchengrößen unter 300 nm auf, sind aber noch nicht völlig optisch klar, insbesondere sind sie empfindlich gegenüber Wirkstoffen aller Art. Der Versuch Wirkstoffe in solche Emulsionen einzuarbeiten, führt zur Vergrößerung der Emulsionströpfchen und zur Trübung des Systems.

Aus DE 44 11 557 Al waren Mikroemulsionen bekannt, deren Ölphase wenigstens teilweise ein Dialkylether mit insgesamt 12 - 24 C-Atomen darstellt. Diese Mikroemulsionen sind zwar sehr feinteilig und optisch klar, jedoch ist auch der Existenzbereich dieser Mikroemulsionen bei Einarbeitung organischer Wirkstoffe sehr begrenzt.

Es bestand daher die Aufgabe, Mikroemulsionen zu entwickeln, deren Existenzbereich breiter und gegenüber eingearbeiteten öllöslichen und wasserlöslichen Wirkstoffen unempfindlicher ist. Es bestand weiterhin die Aufgabe, Emulsionen zu entwickeln, die sich zur Imprägnierung und Ausrüstung flexibler und saugfähiger Trägermaterialien, z.B. von Geweben, Faservliesen oder Schaumstoffen eignen.

Aus WO 97/07195 war zwar schon bekannt, daß sich emulsionsförmige Reiniger mit antimikrobiellen Stoffen, die einen mittleren Teilchendurchmesser von höchstens 1 µm (1000 Nanometer) haben, sich zur Ausrüstung flexibler, saugfähiger Substrate z.B. feuchter Baby-Reinigungstücher eignen. Dort sind aber keine Mikroemulsionen mit Teilchengrößen unter 100 Nanometern offenbart. Je gröber die Tröpfchengröße von Emulsionen bei der Behandlung saugfähiger Substrate ist, desto größer ist auch die Gefahr, daß diese Emulsionen sich durch Filtrations- oder Chromatographie-Effekte auf diesen Substraten trennen und daher die Ölkomponenten und Wirkstoffe sehr ungleichmäßig auf dem Substrat verteilt sind, speziell dann, wenn die Behandlung durch einfaches Tränken des Substrats mit der Emulsion erfolgt.

EP 813 862 A2 offenbart Parfümölkonzentrate in Form von Mikroemulsionen, die einen Dialkylether und ein Alkylglucosid enthalten und zur Ausrüstung von Tüchern, die zur Avivage und Beduftung der Wäsche im Wäschetrockner geeignet sind, verwendet werden können.

Es wurde nunmehr festgestellt, daß sich klare bzw. transparente Mikroemulsionen mit Tröpfchengrößen von weniger als 100 Nanometern besonders gut zur Ausrüstung saugfähiger Substrate eignen. Obwohl prinzipiell alle Mikroemulsionen mit einer mittleren Tröpfchengröße von weniger als 100 Nanometern für diese Anwendung sehr gut geeignet sind, weisen erfindungsgemäße Mikroemulsionen wegen ihrer besonders hohen Stabilität und Unempfindlichkeit gegenüber darin enthaltenen Wirkstoffkomponenten aller Art besondere technische Vorteile für die Ausrüstung saugfähiger Substrate auf.

Ein erster Gegenstand der Erfindung ist die Verwendung von flexiblen und saugfähigen, mit Mikroemulsionen vom Typ Öl-in-Wasser mit einer mittleren Tröpfchengröße unter 100 Nanometern ausgerüsteten Trägern zur Reinigung, Pflege oder dermatologischen Behandlung der Haut oder zur Make-Up-Entfernung, dadurch gekennzeichnet, dass die Mikroemulsionen kosmetische oder dermatologische Wirkstoffe, hautverträgliche Ölkomponenten, von denen wenigstens 30 Gew.-% Dialkylether mit 12 bis 24 C-Atomen, Kohlenwasserstoffe mit 10 bis 32 C-Atomen oder Gemische davon sind, als Emulgatoren Alkyl-(oligo)-glycoside mit linearen Alkylgruppen mit 8 bis 16 C-Atomen und mittleren Oligomerisationsgraden von 1 - 2, sowie als Coemulgatoren lipophile Tenside oder polare Lipide enthalten.
Die Methoden zur Herstellung von Mikroemulsionen bestehen im Prinzip darin, daß man Mischungen aus Wasser, Emulgatoren und Ölkomponente herstellt und die optisch isotropen und thermodynamisch stabilen Existenzbereiche in dem aus diesen Komponenten gebildeten Dreiphasendiagramm ermittelt. Im folgenden werden aber nähere Einzelheiten zur gezielten Herstellung der erfindungsgemäßen Mikroemulsionen gegeben. Als Dialkylether eignen sich sowohl unsymmetrische Ether wie z.B. Cetyl-Methylether oder Lauryl-isobutylether, als auch symmetrische Dialkylether wie z.B. Di-n-Octylether oder Din-Decylether. Auch Ethergemische wie z.B. C₈-C₁₂-Alkyl-, C₈- C₁₀-Alkylether können verwendet werden. Bevorzugt sind aber symmetrische, lineare Dialkylether, insbesondere Di-n-octylether.

Als Kohlenwasserstoffe eignen sich bevorzugt flüssige Kohlenwasserstoffe mit bis zu 32 C-Atomen. Geeignete Kohlenwasserstoffe sind z.B. n-Decan, n-Dodecan, n-Tetradecan, n-Hexadecan, Isohexadecan, 1,3-Di-(2-ethylhexyl)-cyclohexan, Triisobuten, Pentapropylen und andere flüssige Oligoolefine sowie deren Hydrierungsprodukte.

Als hautverträgliche Ölkomponenten können darüber hinaus auch andere, insbesondere in der Kosmetik gebräuchliche Öle, z.B. Fettsäure- oder Fettalkoholester, Triglyceride, verzweigte flüssige Alkohole wie z.B. 2-Octyl-dodecanol oder 2-Hexyl-decanol oder andere flüssige Kohlenwasserstoffe z.B. Paraffinöle oder Squalan enthalten sein. Auch bei 20° C feste, in Kohlenwasserstoffen oder Dialkylethern lösliche Fettstoffe können in geringerem Umfange enthalten sein, solange das Gemisch der enthaltenen Öl- und Fettkomponenten bei 20° C flüssig ist. Solche weiteren, nicht-flüssigen Fettstoffe können z.B. feste Triglyceridfette, Wachse oder Paraffine sein. Insgesamt soll der Anteil an Dialkylethern mit 12 - 24 C-Atomen und/oder Kohlenwasserstoffen mit 10 - 32 C-Atomen jedoch wenigstens 30 Gew.-% der gesamten Öl- und Fettkomponenten ausmachen. Insgesamt sind bevorzugt 0,5 - 10 Gew.-% an hautverträglichen Ölkomponenten in den erfindungsgemäßen Mikroemulsionen enthalten.

Als kosmetische oder dermatologische Wirkstoffe können alle in den genannten Ölkomponenten löslichen Substanzen mit einer günstigen Wirkung auf die Schönheit oder Gesundheit der Haut enthalten sein. Es können aber auch wasserlösliche Wirkstoffe enthalten sein, die eine günstige Wirkung auf den kosmetischen oder den gesundheitlichen Zustand der Haut oder des Bindegewebes haben. Bevorzugt enthalten die erfindungsgemäßen Mikroemulsionen als kosmetische und dermatologische Wirkstoffe öllösliche Wirkstoffe in einer Menge von 1 - 20 Gew.-% der Ölkomponenten oder wasserlösliche Wirkstoffe in einer Konzentration von 0,1 - 10 Gew.-% des Wasseranteils oder beide Arten von Wirkstoffen.

Als öllösliche Wirkstoffe sind solche zu verstehen, die bei 20° C zu wenigstens 1 Gew.-% in der Ölkomponente löslich sind. Geeignete öllösliche kosmetische Wirkstoffe sind z.B. Silikonöle, Ceramid und Ceramidanaloge, Sterine, z.B. Cholesterin oder Phytosterine, öllösliche Vitamine oder Vitamin-Derivate wie z.B. Tocopherol, Tocopherolester, Retinol und Retinolester, Ascorbylpalmitat, Carotine und deren Derivate, γ-Linolensäure und deren Ester, Farnesol, , öllösliche Antioxidantien, öllösliche antimikrobielle Stoffe , öllösliche Pflanzenextrake und physiologisch wirksame Pflanzenöle.

Als wasserlösliche kosmetische Wirkstoffe können z.B. α-Hydroxycarbonsäuren oder deren Salze z.B. Zink-Gluconat, Pyrrolidoncarbonsäure, Ascorbinsäure, Aminosäure, Phytinsäure, Biotin, Rahmnose, Fucose, Panthenol, Pantothenate, Hyaluronsäure und deren Salze, Allantoin , Enzyme, wasserlösliche antimikrobielle Stoffe, z.B. p-Hydroxybenzoesäure, deren Ester und Salze, Natriumsalicylat, 1,6-Hexandiol, 1,5-Pentandiol, Chitosan, Triclosan, Chlorhexidin, Collagen und Proteinhydrolysate, wasserlösliche Extrakte aus Pflanzen und Pflanzenteilen z.B. Samen, Algen, Hefen oder tierischem Gewebe sowie wasserlösliche Deodorantien, z.B. Aluminiumhydroxychlorid oder Alaun.

Bevorzugte Mikroemulsionen gemäß der vorliegenden Erfindung enthalten als öllösliche Wirkstoffe bevorzugt Retinol, Tocopherol oder deren öllösliche Derivate oder öllösliche Ascorbinsäureester, bevorzugt in einer Menge von 0,2 - 2 Gew.-%. Bevorzugte erfindungsgemäße Mikroemulsionen enthalten als wasserlösliche Wirkstoffe bevorzugt wasserlösliche antimikrobielle Stoffe oder wasserlösliche Extrakte aus Pflanzen oder Pflanzenteilen, Algen oder Hefen.

Zur Herstellung der zur Ausrüstung der erfindungsgemäß verwendeten Träger eingesetzten Mikroemulsionen eignen sich alle für die Emulgierung kosmetischer und pharmazeutischer Öle geeigneten, physiologisch und dermatologisch verträglichen Emulgatoren. Bevorzugt verwendet man für diesen Zweck nichtionogene Ethylenoxid-Addukte mit HLB-Werten von 6 - 12. Unter dem HLB-Wert wird dabei ein aus der Struktur errechenbarer Wert gemäß HLB = 0,2 · (100 - L) verstanden, worin L der Anteil der lipophilen Alkyl- oder Acylgruppen in Gewichtsprozent des Gesamtmoleküls ist. Geeignete Emulgatoren sind z.B. die Anlagerungsprodukte von 5 Mol Ethylenoxid an Cetyl- und/oder OleylalkoholGemische (Cetyl-/Oleylalkohol + 5 EO) oder von 3 Mol Ethylenoxid an Lauryl/Myristylalkohol-Gemische (Lauryl-/Myristylalkohol + 3 EO).

Als Coemulgatoren eignen sich hydrophilere Emulgatoren, z.B. vom Typ der nichtionischen Ethylenoxidaddukte mit HLB-Werten oberhalb von 12 oder vom Typ der wasserlöslichen ionischen Tenside.

Besonders vorteilhafte Emulgatoren zur Herstellung der zur Ausrüstung der erfindungsgemäß verwendeten Träger eingesetzten Mikroemulsionen sind Alkyl-(oligo)-glucoside in Kombination mit Coemulgatoren vom Typ der polaren Lipide. Mit dieser Emulgatorkombination lassen sich Mikroemulsionen schon mit einem Emulgatoranteil von weniger als 1 Gewichtsteil Emulgator pro Gewichtsteil der Ölkomponente herstellen.

Alkyl-(oligo)-glucoside sind bekannte oberflächenaktive Stoffe und auf dem Markt unter der Verkaufsbezeichnung Plantaren® 1200 oder Plantaren® 2000 erhältlich. Sie enthalten eine lineare C₈ - C₁₆ - Alkylgruppe glucosidisch an ein Glucosemolekül oder ein oligomeres Glucosemolekül gebunden. Der Oligomerisationsgrad des Glucosidrestes der handelsüblichen Alkyl-(oligo)-glucoside liegt im Mittel zwischen 1 und 2.

Die als Coemulgatoren geeigneten polaren Lipide sind gekennzeichnet durch eine bevorzugt lineare Alkyl- oder Acylgruppe mit 12 - 22 C-Atomen und eine polare Gruppe, z.B. eine Hydroxylgruppe, eine Hydroxypropylgruppe, eine Oxethylgruppe oder eine Dihydroxypropoxygruppe. Geeignete polare Lipide sind z.B. C₁₆-C₂₂-Fettalkohole, Ethylenglycolmonoether von linearen C₁₂-C₁₈-Fettalkoholen oder Fettsäure-(C₁₂-C₂₂)-Monoglyceride oder Sorbitanmonostearate. Auch andere polare Lipide, z.B. Lecithine oder ganz allgemein nichtionogene Emulgatoren mit HLB-Werten unterhalb von 5 (lipophile Tenside) eignen sich als Coemulgatoren.

Bevorzugte zur Ausrüstung der erfindungsgemäß verwendeten Träger eingesetzte Mikroemulsionen enthalten daher als Emulgatoren Alkyl-(oligo)-glucoside mit linearen Alkylgruppen mit 8 - 16 C-Atomen und mittleren Oligomerisationsgraden von 1 - 2 und als Coemulgatoren lipophile Tenside oder polare Lipide. Die Ölkomponenten, Emulgatoren und Coemulgatoren sind darin bevorzugt in einem Gewichtsverhältnis von 1 : (0,2 - 1) : (0,1 - 0,5) enthalten.

Zusätzlich können die zur Ausrüstung der erfindungsgemäß verwendeten Träger eingesetzten Mikroemulsionen noch Hilfsmittel enthalten, die den Geruch, die Lagerstabilität und das Aussehen verbessern. Solche Hilfsmittel sind z.B. Farbstoffe, Duftstoffe, Verdickungsmittel (wasserlösliche Polymere), pH-Stellmittel, Puffersubstanzen und niedere Alkohole, Glycole oder Glycerin.

Die Herstellung der zur Ausrüstung der erfindungsgemäß verwendeten Träger eingesetzten Mikroemulsionen erfordert keine besondere Technik: Ölkomponente, öllösliche Wirkstoffe, Emulgatoren, Coemulgatoren und ggf. öllösliche Hilfsstoffe werden gemischt, gegebenenfalls zur Herstellung einer homogenen Lösung erwärmt und in die erwärmte Lösung werden dann Wasser, das ebenfalls auf die Temperatur der Ölphase erwärmt sein sollte, und nacheinander die wasserlöslichen Hilfsstoffe eingerührt.

Die typischen Einsatzgebiete solcher Träger sind z.B. Tampons und Zellstoffpads sowie Feuchtreinigungstücher zur Hautreinigung, z.B. für die Babypflege oder für die Make-Up-Entfernung. Weitere Einsatzgebiete solcher Träger sind z.B. Gesichtsmasken zur kosmetischen Behandlung der Gesichtshaut oder therapeutische, z.B. transdermal wirkende oder kosmetische Pflaster zur lokalen Langzeit-Hautbehandlung.

Als flexible und saugfähige Träger im Sinne der Erfindung eignen sich z.B. Träger aus Textilfasem, Kollagen oder polymeren Schaumstoffen. Als Textilfasern können sowohl Naturfasern wie z.B. Cellulose (Baumwolle, Leinen), Seide, Wolle, Regeneratcellulose (Viskose, Rayon), Cellulosederivaten als auch synthetische Fasern wie z.B. Polyester-, Polyacrylnitril-, Polyamid- oder Polyolefin-Fasern oder Mischungen solcher Fasern, gewebt oder ungewebt verwendet werden. Diese Fasern können zu saugfähigen Wattepads, Vliesstoffen oder zu Geweben oder Gewirken verarbeitet sein.

Kollagenfasern oder gefriergetrocknete Kollagenschäume sind z.B. als Wirkstoffträger für Gesichtsmasken und Wundauflagen geeignet. Auch flexible und saugfähige polymere Schaumstoffe, z.B. Polyurethanschäume und Polyamidschäume oder andere mikroporöse Polymere sind geeignete Trägersubstrate.

Als saugfähig im Sinne der Erfindung sind solche Trägersubstrate anzusehen, die bei 20° C wenigstens 10 Gew.-%, bezogen auf das Trockengewicht, an Wasser adsorptiv oder kapillar binden können. Bevorzugt eignen sich aber solche Träger, die wenigstens 100 Gew.-% ihres Trockengewichtes an Wasser adsorptiv oder kapillar binden können.

Ein weiterer Erfindungsgegenstand ist das Verfahren zur Herstellung flexibler Artikel zur Reinigung, Pflege oder dermatologischen Behandlung der Haut, bei dem man flexible und saugfähige Träger, bevorzugt aus der Gruppe der Fasermaterialien, mit Mikroemulsionen, enthaltend kosmetische oder dermatologische Wirkstoffe, hautverträgliche Ölkomponenten, von denen wenigstens 30 Gew.-% Dialkylether mit 12 bis 24 C-Atomen, Kohlenwasserstoffe mit 10 bis 32 C-Atomen oder Gemische davon sind, als Emulgatoren Alkyl-(oligo)-glycoside mit linearen Alkylgruppen mit 8 bis 16 C-Atomen und mittleren Oligomerisationsgraden von 1 - 2, sowie als Coemulgatoren lipophile Tenside oder polare Lipide, behandelt und trocknet. Die Behandlung bzw. Ausrüstung der Trägermaterialien mit den erfindungsgemäßen Mikroemulsionen kann nach beliebigen Verfahrensweisen, z.B. durch Aufsprühen, Tauchen und Abquetschen, Durchtränken oder einfach durch Einspritzen der Mikroemulsion in die Trägersubstrate erfolgen. Wenn die erfindungsgemäß ausgerüsteten Träger zur Reinigung der Haut verwendet werden sollen, so kann die Trocknung unterbleiben. Es werden so z.B. Feuchtreinigungstücher oder Feuchtreinigungspads erhalten, die ohne zusätzlichen Wassereinsatz zur Reinigung geeignet sind. Ein besonderer Vorteil der Mikroemulsion gegenüber grobteiligen Emulsionen zeigt sich darin, daß die Mikroemulsion sich aufgrund der Kapillarkräfte gleichmäßig in dem Träger verteilt, ohne daß es zu Inhomogenitäten aufgrund von Filtrations- oder Chromatographie-Effekten kommt. In jedem Fall sind Ölkomponenten und Wirkstoffe gleichmäßig in dem Träger verteilt.

Die folgenden Beispiele sollen den Erfindungsgegenstand näher erläutern:

### Beispiele

Es wurden die in Tabelle I aufgeführten Mikroemulsionen nach folgenden Verfahren hergestellt: Ölkomponenten, Emulgator, Coemulgator und öllösliche Wirkstoffe wurden gemischt und auf 70° C erwärmt. In die erwärmte Lösung wurde auf 30° C erwärmtes Wasser und dann die wasserlöslichen Hilfsstoffe unter Rühren zugegeben. Zuletzt wird das Parfümöl bei 35° C zugegeben. Es bildeten sich spontan klare Mikroemulsionen.

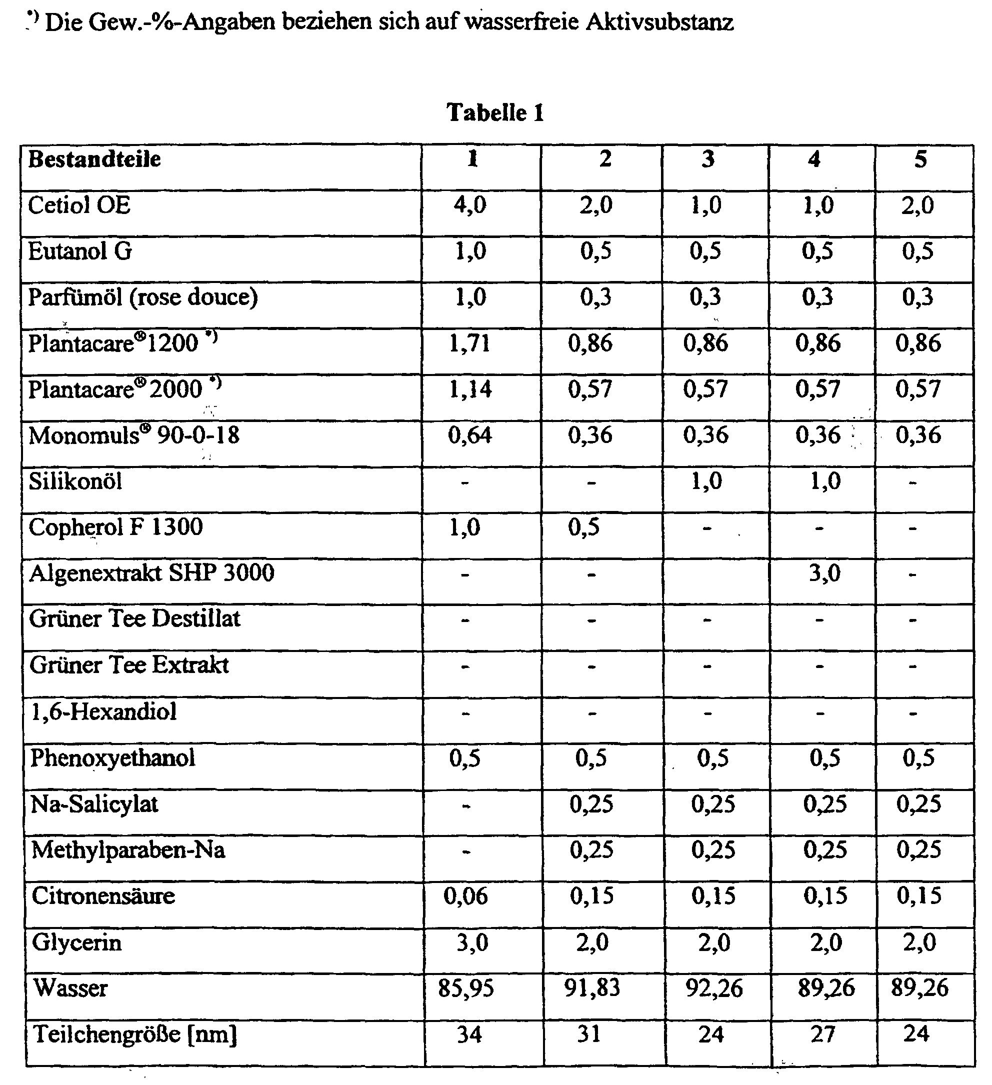

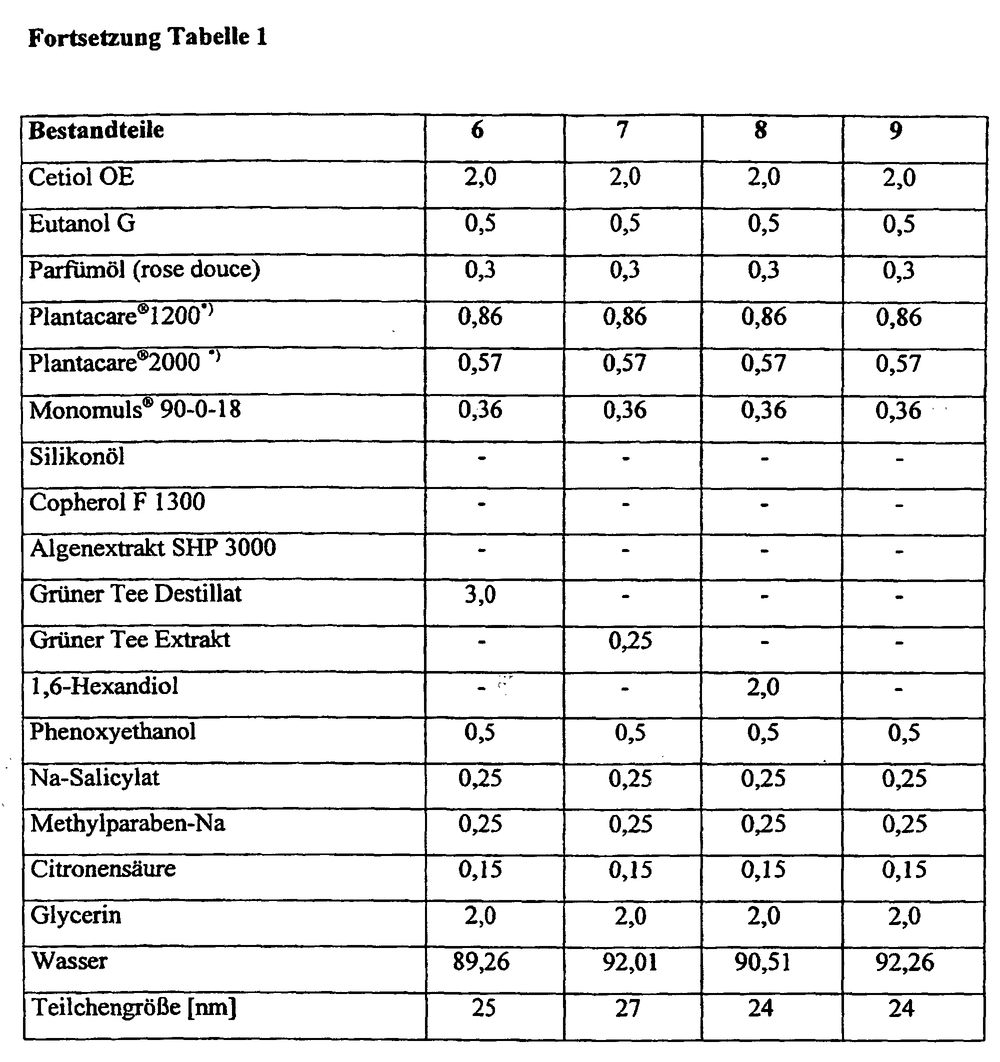

Die Bestimmung der Teilchengröße erfolgte mit Hilfe eines MICROTRAC UPA (Fa. MICROTRAC, ultrakompaktes Feinstkorngranulometer, Modell 3.150)

Es wurden kosmetische Feuchtreinigungstücher für die Hautpflege und zur Make-Up-Entfernung unter Verwendung der Mikroemulsionen gemäß Beispiel 1 - 9 hergestellt. Hierzu wurde 100 g der Mikroemulsion auf 34 g des zu einer Rolle von 4 m aufgewickelten Vliestuches (70 % Viskose, 30 % Polyester) aufgetropft. Das Vliestuch kann auch durch Aufsprühen der Mikroemulsion auf das Tuch vor dem Aufrollen mit einer Menge von ca. 45 g/m² ausgerüstet werden.

Es wurden die folgenden Handelsprodukte eingesetzt:
- Cetiol®OE :: Di-n-octylether
Hersteller: Henkel KGaA
- Eutanol®G :: 2-Octyl-dodecanol
Hersteller: Henkel KGaA
- Plantacare®1200:: C₁₂₋₁₆-Fettalkyloligo-(1,4)-glucosid
(Paste, 50 %ig in Wasser)
Hersteller: Henkel KGaA
- Plantacare®2000 :: C₈₋₁₆-Fettalkyloligo-(1,4)-glucosid
(Lösung, 52 %ig in Wasser)
Hersteller: Henkel KGaA
- Monomuls 90 -0-18 :: Glycerinmonooleat (Monoglyceridgehalt mind 90 Gew.-%,
freies Glycerin max. 2 Gew.-%)
Hersteller: Henkel KGaA (C.F. Grünau)
- Herbasol® Destillat Grüner Tee :: Hersteller: Cosmetochem
- Grüner Tee Extrakt 20747:: Hersteller: Novarom
- Algenextrakt SPH 3000 :: Lieferant: Interorgana
Hersteller: IGV Institut für Getreideverarbeitung GmbH

## Patentansprüche

1. Verwendung von flexiblen und saugfähigen, mit Mikroemulsionen vom Typ ÖI-in-Wasser mit einer mittleren Tröpfchengröße unter 100 Nanometern ausgerüsteten Trägern zur Reinigung, oder Pflege, der Haut oder zur Make-Up-Entfernung, **dadurch gekennzeichnet, dass** die Mikroemulsionen
a) kosmetische oder dermatologische Wirkstoffe,
b) hautverträgliche Ölkomponenten, von denen wenigstens 30 Gew.-% Dialkylether mit 12 bis 24 C-Atomen, Kohlenwasserstoffe mit 10 bis 32 C-Atomen oder Gemische davon sind,
c) als Emulgatoren Alkyl-(oligo)-glycoside mit linearen Alkylgruppen mit 8 bis 16 C-Atomen und mittleren Oligomerisationsgraden von 1- 2, sowie
d) als Coemulgatoren lipophile Tenside oder polare Lipide
enthalten.

2. Verwendung von flexiblen und saugfähigen, mit Mikroemulsionen vom Typ Öl-in-Wasser mit einer mittleren Tröpfchengröße unter 100 Nanometern ausgerüsteten Trägern zur Herstellung einer Zusammensetzung zur dermatologischen Behandlung der Haut, **dadurch gekennzeichnet, dass** die Mikroemulsionen
a) kosmetische oder dermatologische Wirkstoffe,
b) hautverträgliche Ölkomponenten, von denen wenigstens 30 Gew.-% Dialkylether mit 12 bis 24 C-Atomen, Kohlenwasserstoffe mit 10 bis 32 C-Atomen oder Gemische davon sind,
c) als Emulgatoren Alkyl-(oligo)-glycoside mit linearen Alkylgruppen mit 8 bis 16 C-Atomen und mittleren Oligomerisationsgraden von 1- 2, sowie
d) als Coemulgatoren lipophile Tenside oder polare Lipide
enthalten.

3. Verwendung gemäß Anspruch 1 oder 2, **dadurch gekennzeichnet, dass** die Mikroemulsionen öllösliche kosmetische oder dermatologische Wirkstoffe in einer Menge von 1 - 20 Gew.-% der Ölkomponenten oder wasserlösliche kosmetische oder dermatologische Wirkstoffe in einer Menge von 0,1 bis 10 Gew.-% des Wasseranteils enthalten.

4. Verwendung gemäß einem der Ansprüche 1 - 3, **dadurch gekennzeichnet, dass** die öllöslichen kosmetischen oder dermatologischen Wirkstoffe ausgewählt sind aus Silikonölen, Ceramid und Ceramidanalogen, Sterinen, öllöslichen Vitaminen oder Vitaminderivaten, γ-Linolensäure und deren Estern, Farnesol, öllöslichen Antioxidantien, öllöslichen antimikrobiellen Stoffen, öllöslichen Pflanzenextrakten und physiologisch wirksamen Pflanzenölen.

5. Verwendung gemäß einem der Ansprüche 1 - 3, **dadurch gekennzeichnet, dass** die wasserlöslichen kosmetischen oder dermatologischen Wirkstoffe ausgewählt sind aus α-Hydroxycarbonsäuren oder deren Salzen, Pyrrolidoncarbonsäure, Ascorbinsäure, Aminosäure, Phytinsäure, Biotin, Rhamnose, Fucose, Panthenol, Pantothenate, Hyaluronsäure und deren Salzen, Allantoin, Enzymen, wasserlöslichen antimikrobiellen Stoffen, Natriumsalicylat, 1,6-Hexandiol, 1,5-Pentandiol, Chitosan, Triclosan, Chlorhexidin, Collagen, Proteinhydrolysaten, wasserlöslichen Extrakten aus Pflanzen und Pflanzenteilen, wasserlöslichen Extrakten aus Algen oder Hefen oder tierischem Gewebe sowie wasserlöslichen Deodorantien.

6. Verwendung gemäß einem der vorstehenden Ansprüche, **dadurch gekennzeichnet, dass** die in den Mikroemulsionen enthaltenen Ölkomponenten, Emulgatoren und Coemulgatoren im Gewichtsverhältnis 1:(0,2 bis 1): (0,1 bis 0,5) vorliegen.

7. Verwendung gemäß einem der vorstehenden Ansprüche, **dadurch gekennzeichnet, dass** es sich bei dem flexiblen und saugfähigen Träger um eine Gesichtsmaske oder ein Pflaster handelt.

8. Verfahren zur Herstellung flexibler, kosmetischer Hautreinigungs-, kosmetischer Hautpflege- oder dermatologischer Hautbehandlungsartikel, **dadurch gekennzeichnet, dass** dass man flexible, saugfähige Träger, bevorzugt aus der Gruppe der Fasermaterialien, mit Mikroemulsionen vom Typ Öl-in-Wasser mit einer mittleren Tröpfchengröße unter 100 Nanometern, die
a) öllösliche und/oder wasserlösliche kosmetische oder dermatologische Wirkstoffe,
b) hautverträgliche Ölkomponenten, von denen wenigstens 30 Gew.-% Dialkylether mit 12 bis 24 C-Atomen, Kohlenwasserstoffe mit 10 bis 32 C-Atomen oder Gemische davon sind,
c) als Emulgatoren Alkyl-(oligo)-glycoside mit linearen Alkylgruppen mit 8 bis 16 C-Atomen und mittleren Oligomerisationsgraden von 1 - 2, sowie
d) als Coemulgatoren lipophile Tenside oder polare Lipide
enthalten, behandelt und trocknet.

## Claims

1. Use of flexible and absorbent carriers finished with microemulsions of the oil-in-water type with a mean droplet size below 100 nanometres for cleansing or care of the skin or for removing make-up, **characterized in that** the microemulsions contain
a) cosmetic or dermatological active components,
b) skin-compatible oil components of which at least 30% by weight are dialkylethers containing 12 to 24 carbon atoms, hydrocarbons containing 10 to 32 carbon atoms or mixtures thereof,
c) alkyl (oligo)glycosides with linear C₈₋₁₆ alkyl groups and average degrees of oligomerization of 1 to 2 as emulsifiers and
d) lipophilic surfactants or polar lipids as co-emulsifiers.

2. Use of flexible and absorbent carriers finished with microemulsions of the oil-in-water type with a mean droplet size below 100 nanometres for the production of a composition for dermatological treatment of the skin, **characterized in that** the microemulsions contain
a) cosmetic or dermatological active components,
b) skin-compatible oil components of which at least 30% by weight are dialkylethers containing 12 to 24 carbon atoms, hydrocarbons containing 10 to 32 carbon atoms or mixtures thereof,
c) alkyl (oligo)glycosides with linear C₈₋₁₆ alkyl groups and average degrees of oligomerization of 1 to 2 as emulsifiers and
d) lipophilic surfactants or polar lipids as co-emulsifiers.

3. Use claimed in claim 1 or 2, **characterized in that** the microemulsions contain oil-soluble cosmetic or dermatological active components in a quantity of 1 to 20% by weight of the oil components or water-soluble cosmetic or dermatological active components in a quantity of 0.1 to 10% by weight of the water content.

4. Use as claimed in any of claims 1 to 3, **characterized in that** the oil-soluble cosmetic or dermatological active components are selected from silicone oils, ceramide and ceramide analogs, sterols, oil-soluble vitamins or vitamin derivatives, γ-linolenic acid and esters thereof, farnesol, oil-soluble antioxidants, oil-soluble antimicrobial agents, oil-soluble plant extracts and physiologically active vegetable oils.

5. Use claimed in any of claims 1 to 3, **characterized in that** the water-soluble cosmetic or dermatological active components are selected from α-hydroxycarboxylic acids or salts thereof, pyrrolidone carboxylic acid, ascorbic acid, amino acid, phytic acid, biotin, rhamnose, fucose, panthenol, pantothenates, hyaluronic acid and salts thereof, allantoin, enzymes, water-soluble antimicrobial agents, sodium salicylate, hexane-1,6-diol, pentane-1,5-diol, chitosan, triclosan, chlorhexidine, collagen, protein hydrolyzates, water-soluble extracts of plants and plant parts, water-soluble extracts or algae or yeasts or animal tissue and water-soluble deodorants.

6. Use claimed in any of the preceding claims, **characterized in that** the oil components, emulsifiers and co-emulsifiers are present in the microemulsions in a ratio by weight of 1 : (0.2 to 1) : (0.1 to 0.5).

7. Use claimed in any of the preceding claims, **characterized in that** the flexible and absorbent carrier is a face mask or a plaster.

8. Process for the production of flexible, cosmetic skin-cleansing or skin-care or dermatological skin treatment articles, **characterized in that** flexible absorbent carriers, preferably from the group of fibrous materials, are treated with microemulsions of the oil-in-water type with a mean droplet size below 100 nanometres which contain
a) oil-soluble and/or water-soluble cosmetic or dermatological active components,
b) skin-compatible oil components of which at least 30% by weight are dialkylethers containing 12 to 24 carbon atoms, hydrocarbons containing 10 to 32 carbon atoms or mixtures thereof,
c) alkyl (oligo)glycosides with linear C₈₋₁₆ alkyl groups and average degrees of oligomerization of 1 to 2 as emulsifiers and
d) lipophilic surfactants or polar lipids as co-emulsifiers
and dried.

## Revendications

1. Utilisation de supports souples et absorbants, munis de microémulsions de type huile-dans-l'eau, ayant une taille moyenne des gouttelettes inférieure à 100 nanomètres, pour le nettoyage ou le soin de la peau ou pour le démaquillage, **caractérisée en ce que** les microémulsions contiennent
a) des principes actifs cosmétiques ou dermatologiques,
b) des composants huileux compatibles avec la peau, dont au moins 30 % en poids est constitué par des dialkyléthers comprenant 12 à 24 atomes de C, des hydrocarbures comprenant 10 à 32 atomes de C ou des mélanges de ces composés,
c) en tant qu'émulsifiants, des alkyl-(oligo)-glycosides englobant des groupes alkyle linéaires qui comprennent 8 à 16 atomes de C et présentent un degré moyen d'oligomérisation de 1-2, et
d) en tant que co-émulsifiants, des tensioactifs lipophiles ou des lipides polaires.

2. Utilisation de supports souples et absorbants, munis de microémulsions de type huile-dans-l'eau, ayant une taille moyenne des gouttelettes inférieure à 100 nanomètres, pour la préparation d'une composition destinée au traitement dermatologique de la peau, **caractérisée en ce que** les microémulsions contiennent
a) des principes actifs cosmétiques ou dermatologiques,
b) des composants huileux compatibles avec la peau, dont au moins 30 % en poids est constitué par des dialkyléthers comprenant 12 à 24 atomes de C, des hydrocarbures comprenant 10 à 32 atomes de C ou des mélanges de ces composés,
c) en tant qu'émulsifiants, des alkyl-(oligo)-glycosides englobant des groupes alkyle linéaires qui comprennent 8 à 16 atomes de C et présentent un degré moyen d'oligomérisation de 1-2, et
d) en tant que co-émulsifiants, des tensioactifs lipophiles ou des lipides polaires.

3. Utilisation selon la revendication 1 ou 2, **caractérisée en ce que** les microémulsions contiennent des principes actifs cosmétiques ou dermatologiques oléosolubles dans une proportion de 1-20 % en poids par rapport aux composants huileux, ou des principes actifs cosmétiques ou dermatologiques hydrosolubles dans une proportion de 0,1 à 10 % en poids par rapport à la teneur en eau.

4. Utilisation selon l'une quelconque des revendications 1 à 3, **caractérisée en ce que** les principes actifs cosmétiques ou dermatologiques oléosolubles sont choisis parmi les huiles de silicone, les céramides et les analogues de céramides, les stérines, les vitamines ou dérivés de vitamines oléosolubles, l'acide γ-linolénique et ses esters, le farnesol, les anti-oxydants oléosolubles, les substances anti-microbiennes oléosolubles, les extraits de plantes oléosolubles et les huiles végétales physiologiquement actives.

5. Utilisation selon l'une quelconque des revendications 1 à 3, **caractérisée en ce que** les principes actifs cosmétiques ou dermatologiques hydrosolubles sont choisis parmi les acides α-hydroxycarboxyliques ou leurs sels, l'acide pyrrolidonecarboxylique, l'acide ascorbique, les acides aminés, l'acide phytique, la biotine, le rhamnose, le fucose, le panthénol, les pantothénates, l'acide hyaluronique et ses sels, l'allantoïne, les enzymes, les substances anti-microbiennes hydrosolubles, le salicylate de sodium, le 1,6-hexanediol, le 1,5-pentanediol, le chitosan, le triclosan, la chlorhexidine, le collagène, les hydrolysats de protéines, les extraits des plantes et parties de plantes hydrosolubles, les extraits d'algues ou de levures ou de tissus animaux hydrosolubles et les déodorants hydrosolubles.

6. Utilisation selon l'une quelconque des revendications précédentes, **caractérisée en ce que** les composants huileux, les émulsifiants et les co-émulsifiants contenus dans les microémulsions sont présents dans un rapport en poids de 1 : (0,2 à 1) : (0,1 à 0,5).

7. Utilisation selon l'une quelconque des revendications précédentes, **caractérisée en ce que** le support souple et absorbant est un masque facial ou un emplâtre.

8. Procédé de préparation d'un article souple destiné au nettoyage de la peau, au soin cosmétique de la peau ou au traitement dermatologique de la peau, **caractérisé en ce que** l'on traite et sèche des supports souples et absorbants, appartenant de préférence au groupe des matériaux fibreux, munis de microémulsions de type huile-dans-l'eau, ayant une taille moyenne des gouttelettes inférieure à 100 nanomètres, qui contiennent
a) des principes actifs cosmétiques ou dermatologiques oléosolubles et/ou hydrosolubles,
b) des composants huileux compatibles avec la peau, dont au moins 30 % en poids est constitué par des dialkyléthers comprenant 12 à 24 atomes de C, des hydrocarbures comprenant 10 à 32 atomes de C ou des mélanges de ces composés,
c) en tant qu'émulsifiants, des alkyl-(oligo)-glycosides englobant des groupes alkyle linéaires qui comprennent 8 à 16 atomes de C et présentent un degré moyen d'oligomérisation de 1-2, et
d) en tant que co-émulsifiants, des tensioactifs lipophiles ou des lipides polaires.
